# FASCICULE DE BREVET EUROPEEN

(11) **EP 2 176 214 B1**
(45) Date de publication et mention de la délivrance du brevet: **05.03.2014**
(21) Numéro de dépôt: 08842551.7
(22) Date de dépôt: 01.08.2008
(51) Int. Cl.: C07C 231/02, C07C 253/14, C07C 233/18, C07C 255/37

(54) **NOUVEAU PROCEDE DE SYNTHESE DU (7-METHOXY-1-NAPHTYL)ACETONITRILE ET APPLICATION A LA SYNTHESE DE L'AGOMELATINE**
NEUES VERFAHREN ZUR SYNTHESIS VON (7-METHOXY-1-NAPHTHYL)ACETONITRIL UND SEINE ANWENDUNG BEI DER SYNTHESE VON AGOMELATIN
NOVEL METHOD FOR THE SYNTHESIS OF (7-METHOXY-1-NAPHTHYL)ACETONITRILE AND APPLICATION IN THE SYNTHESIS OF AGOMELATINE

(30) Priorité: 03.08.2007 FR 0705688
(43) Date de publication de la demande: 21.04.2010
(73) Titulaire: Les Laboratoires Servier, 92284 Suresnes Cedex (FR)
(72) Inventeur: DUBUFFET, Thierry, F-76190 Autretot (FR); LECOUVE, Jean-Pierre, F-76600 Le Havre (FR); HERMET, Jean-Paul, 74200 THONON-LES-BAINS (FR)
(86) Numéro de dépôt international: PCT/FR2008/001146
(87) Numéro de publication internationale: WO 2009/053545

(56) Documents cités:
- EP-A- 0 709 371
- EP-A- 1 564 202
- SCHREIBER, KURT C. ET AL: "Conjugation in the naphthalene series. II. Solvolysis of x-methoxy-y-bromomethylnaphthalenes" JOURNAL OF THE AMERICAN CHEMICAL SOCIETY , 84, 859-62 CODEN: JACSAT; ISSN: 0002-7863, 1962, XP002465455
- ARROWSMITH, G.B. ET. AL.: "Physical properties and chemical constitution. Part XLI. Naphthalene compounds" JOURNAL OF THE CHEMICAL SOCIETY, 1965, pages 2072-2078, XP002465456

## Description

La présente invention concerne un procédé de synthèse industriel du (7-méthoxy-1-naphtyl)acétonitrile, et son application à la production industrielle de l'agomélatine ou *N-*[2-(7-méthoxy-1-naphtyl)éthyl]acétamide.

Plus spécifiquement, la présente invention concerne un procédé de synthèse industriel du composé de formule (I) :

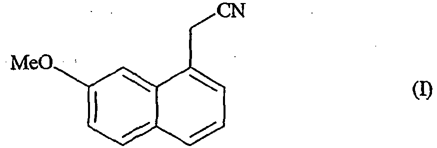

Le composé de formule (I) obtenu selon le procédé de l'invention est utile dans la synthèse de l'agomélatine ou N-[2-(7-méthoxy-1-naphtyl)éthyl]acétamide de formule (II) :

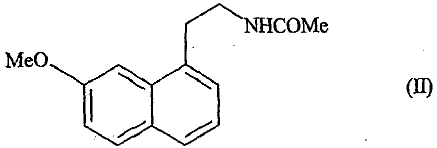

L'agomélatine ou N-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans le brevet européen EP 0 447 285, ainsi que dans la demande de brevet EP 1 564 202.

Compte-tenu de l'intérêt pharmaceutique de ce composé, il est important de pouvoir y accéder avec un procédé de synthèse industriel performant, facilement transposable à l'échelle industrielle, conduisant à l'agomélatine avec un bon rendement, et une excellente pureté, à partir de matières premières bon marché et aisément accessibles.

Le brevet EP 0 447 285 décrit l'accès en huit étapes à l'agomélatine à partir de la 7-méthoxy-1-tétralone avec un rendement moyen inférieur à 30%. En particulier, l'accès au (7-méthoxy-1-naphtyl)acétonitrile implique six étapes réctionnelles et, transposé à l'échelle industrielle, il a rapidement été mis en évidence des difficultés de mise en oeuvre de ce procédé.

La littérature décrit l'accès en trois étapes au (7-méthoxy-1-naphtyl)acétonitrile à partir de la 7-méthoxy-1-tétralone par action de LiCH₂CN suivie d'une déshydrogénation au DDQ (2,3-dichloro-5,6-dicyano-1,4-benzoquinone) et enfin d'une déshydratation en milieu acide (Synthetic Communication, 2001, 31(4), 621-629). Toutefois le rendement global est moyen (76%) et surtout le DDQ utilisé dans la réaction de déshydrogénation ainsi que le reflux de benzène nécessaire à la troisième étape ne répondent pas aux contraintes industrielles de coût et d'environnement.

La demande de brevet EP 1 564 202 décrit un procédé industriel particulièrement avantageux ne nécessitant que quatre étapes à partir de la 7-méthoxy-1-tétralone, dont deux étapes pour accéder au (7-méthoxy-1-naphtyl)acétonitrile, avec un rendement global moyen supérieur à 60%.

Si diverses synthèses de l'art antérieur décrivent l'accès au (7-méthoxy-1-naphtyl)acétonitrile, la plupart utilise comme matière première la 7-méthoxy-1-tétralone, qui est un composé onéreux dont la synthèse est délicate. Ainsi l'élaboration de nouvelles voies de synthèse simples et reproductibles impliquant d'autres matières premières reste une nécessité toujours d'actualité.

La demanderesse a présentement mis au point une nouvelle synthèse industrielle qui conduit, de façon reproductible et sans nécessiter de purification laborieuse, à l'agomélatine avec une pureté qui est compatible avec son utilisation comme principe actif pharmaceutique, à partir d'une matière première moins onéreuse et plus facilement accessible.

En particulier, la demanderesse a présentement mis au point un nouveau procédé de synthèse industriel permettant d'obtenir le (7-méthoxy-1-naphtyl)acétonitrile de façon reproductible et sans nécessiter de purification laborieuse, en utilisant comme matière première l'acide 7-méthoxy-1-naphtoïque. L'accès à l'acide 7-méthoxy-1-naphtoïque est décrit dans l'art antérieur à partir de l'anisole par D. Becker et al. avec d'excellents rendements : Tetrahedron Letters, 27 (32), 3775-3776, 1986.

Plus spécifiquement, la présente invention concerne un procédé de synthèse industriel du composé de formule (I) : 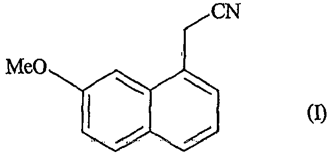 caractérisé en ce que l'on met en réaction l'acide 7-méthoxy-1-naphtoïque de formule (III): 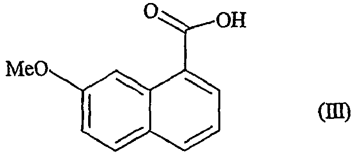 que l'on soumet à l'action d'un agent réducteur pour conduire au composé de formule (IV): 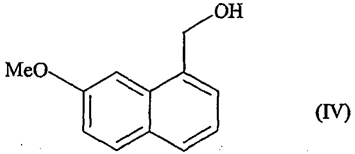 composé de formule (IV) dont la fonction OH est transformée en groupe partant, et que l'on met en réaction avec un réactif de cyanation pour conduire au composé de formule (I) que l'on isole sous la forme d'un solide.

Parmi les agents réducteurs utilisables pour la réduction de l'acide de formule (III) dans le procédé de la présente invention, on peut citer à titre non limitatif BH₃, NaBH₄/AlCl₃, 9-borabicyclo[3.3.1]nonane, LiAlH₄, AlH₃, l'hydrure de diisobutylaluminium (DIBAL-H), et plus particulièrement BH₃-THF.

La réduction de l'acide de formule (III) dans le procédé de la présente invention peut également être avantageusement réalisée après transformation de l'acide de formule (III) en dérivé d'acide et plus particulièrement en ester, puis réduction de ce dérivé d'acide en alcool de formule (IV) en utilisant les agents réducteurs LiAlH₄, NaBH₄ associé ou non à MeOH, LiBH₄, DIBAL-H, hydrure de bis(2-methoxyethoxy)aluminium sodium (Red-Al).

Avantageusement, le groupement OH du composé de formule (IV) du procédé de la présente invention est transformé en halogène et plus particulièrement le brome ou le chlore, ou en groupement tosylate, mésylate, nosylate ou triflate. Plus préférentiellement, la transformation en groupe partant est réalisée par le tribromure de bore, le chlorure de thionyle, le chlorure de mésyle, le chlorure de tosyle, le chlorure de trifluorométhanesulfonyle ou le chlorure de nosyle. Avantageusement le groupement OH du composé de formule (IV) est transformé en chlore par l'action du chlorure de thionyle.

Parmi les réactifs de cyanation utilisables dans le procédé de la présente invention, on peut citer à titre non limitatif le cyanure de triméthylsilyle, le cyanure de lithium, le cyanure de sodium, le cyanure de potassium et le cyanure de tétrabutylammonium. Le réactif de cyanation préféré est le cyanure de potassium.

Le composé de formule (I) ainsi obtenu est, le cas échéant, soumis à une réduction puis à une réaction de couplage avec l'anhydride acétique pour conduire à l'agomélatine.

### Exemple : (7-Méthoxy-1-naphtyl)acétonitrile

### Stade A : (7-Méthoxy-1-napthyl)méthanol

A 1 équivalent d'acide 7-méthoxy-1-naphtoïque (100 g) dans le THF (3,8 ml/g) sont ajoutés lentement 2,5 équivalents de BH₃-THF 1M (1,235 1). Durant l'addition, la température du milieu réactionnel est maintenue à 5°C (+/- 2°C) puis le milieu est laissé à température ambiante pendant 3 heures. Après évaporation sous pression réduite, le résidu est repris par du dichlorométhane et lavé à l'eau. La phase organique est séchée sur MgSO4, puis les solvants évaporés sous pression réduite pour conduire au produit du titre sous la forme d'un solide marron clair avec un rendement de 91%.
*Point de fusion: 72-74°C*

### Stade B : 1-(Chlorométhyl)-7-méthoxynaphtalène

A une solution du produit obtenu au stade A (100 g) dans le dichlorométhane (5ml/g), sont ajoutés 2 équivalents de chlorure de thionyle (126,35 g). Le milieu est porté et maintenu au reflux pendant 2 heures. Après retour à température ambiante, le solvant est évaporé sous pression réduite. L'huile obtenue est reprise par de l'acétate d'éthyle et lavée par de l'eau puis par une solution saturée de NaCl. Le solvant est évaporé sous pression réduite et le brut nettoyé par passage sur un bouchon de silice (éluant: heptane). Le produit du titre est obtenu sous la forme d'un solide jaune avec un rendement de 84%.

### Stade C : (7-Méthoxy-1-naphtyl)acétonitrile

A une solution du produit obtenu au stade B (100 g) dans 30 ml/g de DMSO et 5 ml/g d'eau sont ajoutés 1,2 équivalents de cyanure de potassium (37,8 g). Le milieu réactionnel est porté et maintenu à 65°C pendant 3 heures. Après retour à température ambiante, un binaire MTBE/eau (1/1) est ajouté au milieu. La phase aqueuse est éliminée. La phase organique est lavée plusieurs fois par de l'eau puis par une solution saturée de NaCl. Les solvants sont distillés et le produit du titre est obtenu avec un rendement quantitatif.
*Point de fusion : 83°C*

## Revendications

1. Procédé de synthèse industriel du composé de formule (I) 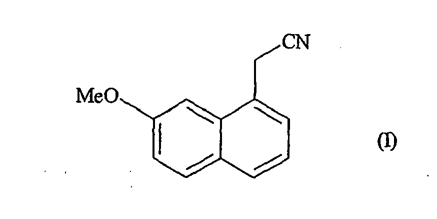 **caractérisé en ce que** l'on met en réaction l'acide 7-méthoxy-1-naphtoïque de formule (III): 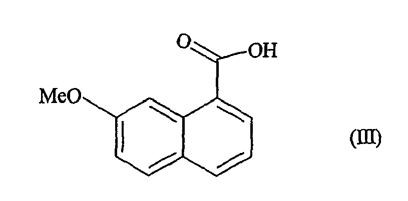 que l'on soumet à l'action d'un agent réducteur pour conduire au composé de formule (IV): 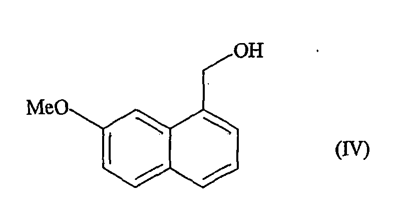 composé de formule (IV) dont la fonction OH est transformée en groupe partant, et que l'on met en réaction avec un réactif de cyanation pour conduire au composé de formule (I) que l'on isole sous la forme d'un solide.

2. Procédé de synthèse du composé de formule (I) selon la revendication 1 **caractérisé en ce que** la réduction de l'acide de formule (III) est réalisée avec BH₃-THF.

3. Procédé de synthèse du composé de formule (I) selon la revendication 1, **caractérisé en ce que** le groupement OH du composé de formule (IV) est transformé en chlore par l'action du chlorure de thionyle.

4. Procédé de synthèse du composé de formule (I) selon la revendication 1, **caractérisé en ce que** le réactif de cyanation utilisé est le cyanure de potassium.

## Patentansprüche

1. Verfahren zur industriellen Synthese der Verbindung der Formel (I) 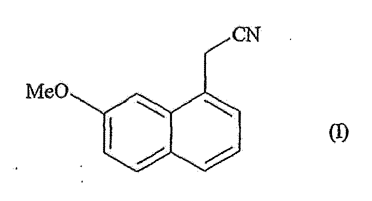 **dadurch gekennzeichnet, dass** man 7-Methoxy-1-naphthoesäure der Formel (III): 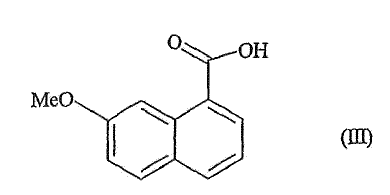 der Einwirkung eines Reduktionsmittels unterwirft zur Bildung der Verbindung der Formel (IV): 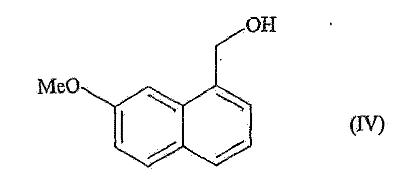 die OH-Funktion der Verbindung der Formel (IV) in eine austretende Gruppe umwandelt und mit einem Cyanierungsreagens umsetzt zur Bildung der Verbindung der Formel (I), welche man in Form eines Feststoffs isoliert.

2. Verfahren zur Synthese der Verbindung der Formel (I) nach Anspruch 1, **dadurch gekennzeichnet, dass** man die Reduktion der Säure der Formel (III) mit BH₃-THF bewirkt.

3. Verfahren zur Synthese der Verbindung der Formel (I) nach Anspruch 1, **dadurch gekennzeichnet, dass** man die OH-Gruppe der Verbindung der Formel (IV) durch Einwirkung von Thionylchlorid in Chlor umwandelt.

4. Verfahren zur Synthese der Verbindung der Formel (I) nach Anspruch 1, **dadurch gekennzeichnet, dass** das verwendete Cyanierungsreagens Kaliumcyanid ist.

## Claims

1. Process for the industrial synthesis of the compound of formula (I) 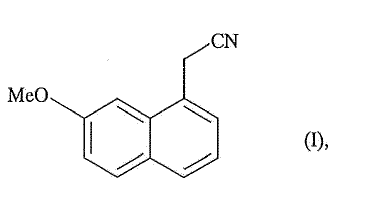 **characterised in that** 7-methoxy-1-naphthoic acid of formula (III) is used for the reaction: 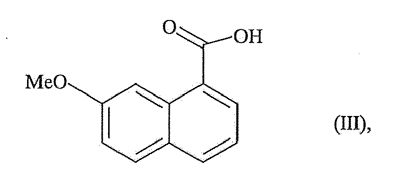 which is subjected to the action of a reducing agent to yield the compound of formula (IV): 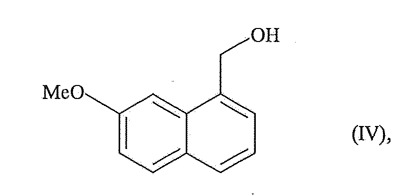 the OH function of which compound of formula (IV) is converted into a leaving group and which is reacted with a cyanation reagent to yield the compound of formula (I), which is isolated in the form of a solid.

2. Process for the synthesis of the compound of formula (I) according to claim 1, **characterised in that** the reduction of the acid of formula (III) is carried out using BH₃-THF.

3. Process for the synthesis of the compound of formula (I) according to claim 1, **characterised in that** the OH group of the compound of formula (IV) is converted into chlorine by the action of thionyl chloride.

4. Process for the synthesis of the compound of formula (I) according to claim 1, **characterised in that** the cyanation reagent used is potassium cyanide.
